# EUROPEAN PATENT SPECIFICATION

(11) **EP 2 025 763 B1**
(45) Date of publication and mention of the grant of the patent: **26.01.2011**
(21) Application number: 08162382.9
(22) Date of filing: 14.08.2008
(51) Int. Cl.: C12Q 1/68

(54) **Nucleic acid amplification method**
Verfahren zur Amplifikation von Nukleinsäure
Procédé d'amplification d'acide nucléique

(30) Priority: 15.08.2007 JP 2007211633
(43) Date of publication of application: 18.02.2009
(73) Proprietor: FUJIFILM Corporation, Tokyo 106-0031 (JP)
(72) Inventor: Miyoshi, Hayato, Ashigarakami-gun Kanagawa 258-8577 (JP); Iwaki, Yoshihide, Ashigarakami-gun Kanagawa 258-8577 (JP); Mori, Toshihiro, Ashigarakami-gun Kanagawa 258-8577 (JP)
(74) Representative: HOFFMANN EITLE

(56) References cited:
- EP-A- 1 837 408
- JP-A- 2004 154 008
- JP-A- 2004 257 901
- US-A- 6 033 881
- NOTOMI T ET AL: "Loop-mediated isothermal amplification of DNA" NUCLEIC ACIDS RESEARCH, OXFORD UNIVERSITY PRESS, SURREY, GB, vol. 28, no. 12, 15 June 2000 (2000-06-15), pages 1362-4962, XP002292090 ISSN: 0305-1048
- KITAO S ET AL: "Detecting nucleic acid, by performing loop-mediated isothermalamplification of target sequence of nucleic acid, obtaining nucleic acid of repeated structures, irradiating nucleic acid and detecting target sequence using scattered light" WPI/THOMSON,, vol. 2004, no. 65, 16 September 2004 (2004-09-16), XP002499810
- BABA Y ET AL: "Detecting nucleic acid such as DNA, cDNA and RNA, involves performing nucleic acid amplification in microchip under isothermal conditions, and analyzing obtained amplified product on microchip" WPI/THOMSON,, vol. 2004, no. 42, 3 June 2004 (2004-06-03), XP002499809

## Description

### Technical Field

The present invention relates to a nucleic acid amplification method. More specifically, the present invention relates to a nucleic acid amplification method that comprises performing a polymerase reaction through incubation of a reaction solution using DNA polymerase.

### Background Art

In molecular biological research, nucleic acid amplification is generally performed by an enzymatic method using DNA polymerase. Polymerase chain reaction (PCR) is broadly known as a nucleic acid amplification method. For amplification of a target nucleic acid sequence, the PCR method comprises the three steps of: denaturing (denaturation step) double-stranded DNA as a template into single-stranded DNAs; annealing (annealing step) primers to the single-stranded DNAs; and elongating (elongation step) complementary strands using the primers as origins. According to a general PCR method, the denaturation step, the annealing step, and the elongation step are each performed at different temperatures using a thermal cycler.. However, implementation of nucleic acid amplification reactions at three different types of temperature is problematic in that temperature control is complicated and time loss increases in proportion to the number of cycles.

Hence, nucleic acid amplification methods that can be performed under isothermal conditions have been developed. Examples of such methods include An RCA (Rolling Circle Amplification: Proc. Natl. Acad. Sci, vol. 92, 4641-4645 (1995)), ICAN (Isothermal and Chimeric primer-initiated Amplification of Nucleic acids), LAMP (Loop-Mediated Isothermal Amplification of DNA; Bio Industry, vol. 18, No. 2 (2001)), NASBA (Nucleic acid Sequence-based Amplification method; Nature, 350,91 - (1991)), and TMA (Transcription mediated amplification method; J. Clin Microbiol. Vol. 31, 3270 - (1993)).

An SDA method (JP Patent Publication (Kokai) No. 5-130870 A (1993)) is a cycling assay method using DNA polymerase with strand displacement activity and restriction enzyme, which is a method for amplifying a target site of a target nucleic acid fragment using a polymerase elongation reaction. This method comprises performing a polymerase elongation reaction using primers (as origins) that have specifically hybridized to target sites of target nucleic acid fragments and , while causing restriction enzyme to act thereon, so as to digest the elongated products into two parts. The 5' side parts of the elongated products work as new primers, so that another elongation reaction proceeds again with the use of DNA polymerase with strand displacement activity. Again, restriction enzyme act on the elongated products and another elongation reaction proceeds. Such an elongation reaction with the use of polymerase and such a digestion reaction with the use of restriction enzyme are repeated periodically in order. Here, the elongation reaction with the use of polymerase and the digestion reaction with the use of restriction enzyme can be implemented under isothermal conditions. However, the use of restriction enzyme in addition to polymerase is required, and thus the method is expensive and the design of primers should be improved.

A LAMP method is a method for amplifying target sites of a target nucleic acid fragment that has been developed in recent years. This method is a method for amplifying target sites of a target nucleic acid fragment as special structure which is complementary to the elongated region from the 3' terminal by 5' terminal of the primer, under isothermal conditions through the use of at least four types of primer that complementarily recognize at least six specific sites of a target nucleic acid fragment and strand-displacement-type Bst DNA polymerase lacking 5'→3' nuclease activity and catalyzing an elongation reaction while liberating double-stranded DNA on the template in the form of single-stranded DNAs. However, the method requires the use of at least four types of primer that recognize six specific sites, so that the design of primers is very difficult.

An ICAN method is a method for amplifying target sites of a target nucleic acid fragment that has been developed in recent years. The ICAN method is an isothermal gene amplification method using RNA-DNA chimeric primers, DNA polymerase having strand displacement activity and template exchange activity, and RNaseH. After chimeric primers bind to a template, a complementary strand is synthesized by DNA polymerase. Subsequently, RNaseH cleaves RNA portions derived from the chimeric primers and then an elongation reaction accompanied by a strand displacement reaction and a template exchange reaction takes place repeatedly from the cleaved sites, so that the gene amplification is performed. However, this method also requires the use of special primers that are chimeric primers and thus the design of such primers is very difficult.

JP Patent Publication (Kohyo) No. 11-509406 A discloses an amplification method, by which, in the presence of DNA polymerase capable of strand displacement, DNA within a target region is amplified by an isothermal reaction using at least a set of oligonucleotide primers. However, the method disclosed in JP Patent Publication (Kohyo) No. 11-509406 A is problematic in that it requires a relatively long reaction time, for example. Therefore, it has been desired to develop a nucleic acid amplification method that can be conveniently implemented isothermally via simple primer design, as with the PCR method.

### Disclosure of the Invention

An object to be achieved by the present invention is to provide a nucleic acid amplification method by which a nucleic acid can be amplified using oligonucleotide primers and DNA polymerase. Furthermore, an object to be achieved by the present invention is to provide a nucleic acid amplification method by which a target nucleic acid sequence can be amplified in a short time at a high efficacy and a target nucleic acid sequence can be specifically amplified.

As a result of intensive studies to achieve the above objects, the present inventors have discovered that a nucleic acid fragment can be efficiently amplified within a short time by designing a first oligonucleotide primer and a second oligonucleotide primer in such a way that any one of the following requirements is satisfied.
(1) As to the region which contains two identical sequences X of serial 4 or more nucleotides within the region of 200 or less nucleotides, a first oligonucleotide primer is designed in a region which is sandwiched by a 5' terminal nucleotide of the 5'-slide sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

Specifically, the present invention provides a nucleic acid amplification method according to the claims which comprises performing incubation of a reaction solution containing at least one type of deoxynucleotide triphosphate, at least one type of DNA polymerase having strand displacement activity, at least two types of oligonucleotide primer, and the nucleic acid fragment as a template so as to perform a polymerase reaction that initiates from the 3' end of the primer and thus amplifying the nucleic acid fragment, wherein a first oligonucleotide primer and a second oligonucleotide primer are designed in such a way that a region which contains two identical sequences X of serial 4 or more nucleotides within the region of 200 or less nucleotides, or a part thereof can be amplified.

The present invention is characterized in any of the followings.
(1) As to the region which contains two identical sequences X of serial 4 or more nucleotides within the region of 200 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side, sequence X, and the second oligonucleotide primer is designed at a 5' side of a sequence which is complementary to the first oligonucleotide primer.
(2) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

In the present invention, "an oligonucleotide primer is designed in a region A" means that an oligonucleotide primer having a substantially identical sequence to the region A is designed.

In other words, "an oligonucleotide primer is designed in a region A" means that an oligotueleotide primer which can anneal to a complementary sequence of the region A, namely which is substantially complementary to the complementary sequence of the region A, is designed.

In the present invention, "a region which is within 100 nucleotides at a 5' side of the sequence X" means a region which is sandwiched by a position of nucleotide which is apart from 5' side of the sequence X by one nucleotide and a position of nucleotide which is apart from 5' side of the sequence X by 100 nucleotides (the position of nucleotide which is apart from 5' side of the sequence X by one nucleotide and the position of nucleotide which is apart from 5' side of the sequence X by 100 nucleotides are included in this region.).

In the present invention, "a region which is within 100 nucleotides at a 3' side of the sequence X" means a region which is sandwiched by a position of nucleotide which is apart from 3' side of the sequence X by one nucleotide and a position of nucleotide which is apart from 3' side of the sequence X by 100 nucleotides (the position of nucleotide which is apart from 3' side of the sequence X by one nucleotide and the position of nucleotide which is apart from 3' side of the sequence X by 100 nucleotides are included in this region.).

Preferably, the sequence X and a sequence Xc which is complementary to the sequence X, comprise 5 or more nucleotides.

Preferably, the sequence X and a sequence Xc which is complementary to the sequence X, comprise 7 or more nucleotides.

Preferably, the reaction solution contains at least 0.05% or more surfactant.

Preferably, the surfactant is a nonionic surfactant.

Preferably, the nonionic surfactant is selected from among a polyoxyethylene sorbitan fatty acid ester-based surfactant, a polyoxyethylene alkyl phenol ether-based surfactant, and a polyoxyethylene alkyl ether-based surfactant.

Preferably, the reaction solution contains a divalent cation.

Preferably, the divalent cation is magnesium ion.

Preferably, the reaction solution further contains a melting temperature adjusting agent.

Preferably, the melting temperature adjusting agent is dimethyl sulfoxide, betaine, formamide, or glycerol, or a mixture of two or more types thereof.

Preferably, the reaction solution contains each deoxynucleotide triphosphate of 1.0 mM to 100 mM.

Preferably, the reaction solution contains each oligonucleotide primer of 1µM to 100µM.

Preferably, the oligonucleotide primers are substantially complementary to portions of the template nucleic acid fragment.

Preferably, only the 3' terminal region of the oligonucleotide primers is substantially complementary to portions of the template nucleic acid fragment.

Preferably, the oligonucleotide primers are substantially complementary to only consecutive 1 site of the template nucleic acid fragment

Preferably, at least one type of the DNA polymerase having strand displacement activity is polymerase selected from the group consisting of *Bacillus stearothermophilus*-derived 5'→3' exonuclease-deficient Bst. DNA polymerase, *Bacillus caldotenax*-derived 5'→3'exonuclease-deficient Bca DNA polymerase, and *Thermococcus litoralis*-derived 5'→3' exonuclease-deficient Vent. DNA polymerase.

Preferably, a step of amplifying the nucleic acid fragment is carried out substantially isothermally.

Preferably, a step of amplifying the nucleic acid fragment is carried out at a temperature of a room temperature to 100°C.

According to the present invention, amplified products are successively extended, and thus a target nucleic acid sequence can be amplified at extremely high amplification efficiency.

### Brief Description of the Drawings

Fig. 1 shows details of the positional relationship of the primers used in the example to the β-actin gene.
Fig. 2 shows the results of fluorescence detection of the amplified products obtained by the amplification reaction of the present invention.
Fig. 3 shows the results of the electrophoresis of the amplified products obtained by the amplification reaction of the present invention.
Fig. 4 shows details of the positional relationship of the primers used in the example to the β3AR gene.
Fig. 5 shows the results of fluorescence detection of the amplified product obtained by the amplification reaction of the comparative example.
Fig. 6 shows the results of the electrophoresis of the amplified products obtained by the amplification reaction of the present invention.
Fig. 7 shows details of the positional relationship of the primers used in the example to chromosome 7.
Fig. 8 shows the results of fluorescence detection of the amplified product obtained by the amplification reaction of the comparative example.
Fig. 9 shows the results of the electrophoresis of the amplified products obtained by the amplification reaction of the present invention.
Fig. 10 shows details of the positional relationship of the primers used in the example to chromosome 7.
Fig. 11 shows the results of fluorescence detection of the amplified product obtained by the amplification reaction of the comparative example.
Fig. 12 shows the results of the electrophoresis of the amplified products obtained by the amplification reaction of the present invention.
Fig. 13 shows the outline (first half) of the first embodiment of the nucleic acid amplification method of the present invention.
Fig. 14 shows the outline (last half) of the first embodiment of the nucleic acid amplification method of the present invention.
Fig. 15 shows the outline (first half) of the second embodiment of the nucleic acid amplification method of the present invention.
Fig. 16 shows the outline (last half) of the second embodiment of the nucleic acid amplification method of the present invention.
Fig. 17 shows the outline (first half) of the third embodiment of the nucleic acid amplification method of the present invention.
Fig. 18 shows the outline (last half) of the third embodiment of the nucleic acid amplification method of the present invention.

### Preferred Embodiments of the Invention

The present invention will be further described in detail as follows.

The nucleic acid amplification method of the present invention comprises performing incubation of a reaction solution containing at least one type of deoxynucleotide triphosphate, at least one type of DNA polymerase having strand displacement activity, at least two types of oligonucleotide primer, and the nucleic acid fragment as a template so as to perform a polymerase reaction that initiates from the 3' end of the primer and thus amplifying the nucleic acid fragment, 0wherein a first oligonucleotide primer and a second oligonucleotide primer are designed in such a way that a region which contains two identical sequences X of serial 4 or more nucleotides within the region of 200 or less nucleotides, or a part thereof can be amplified.

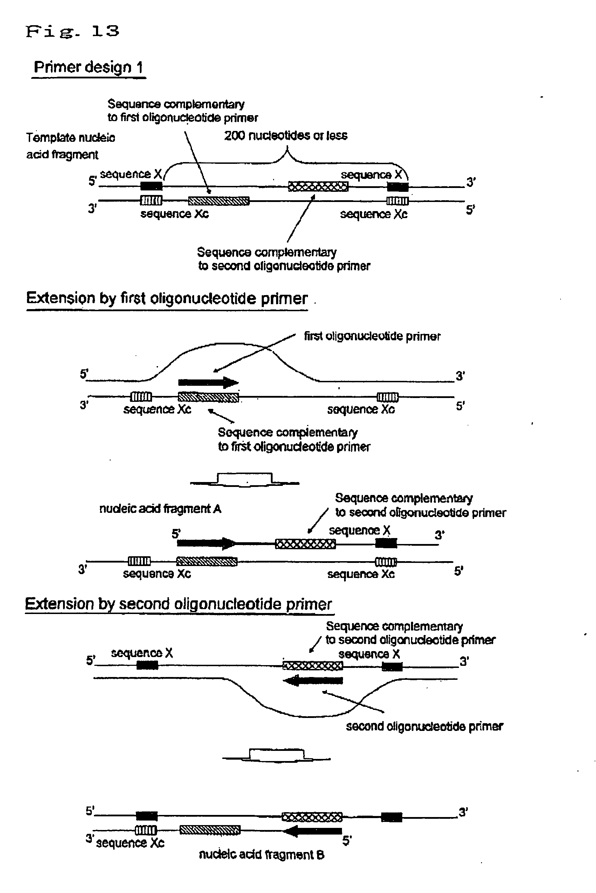

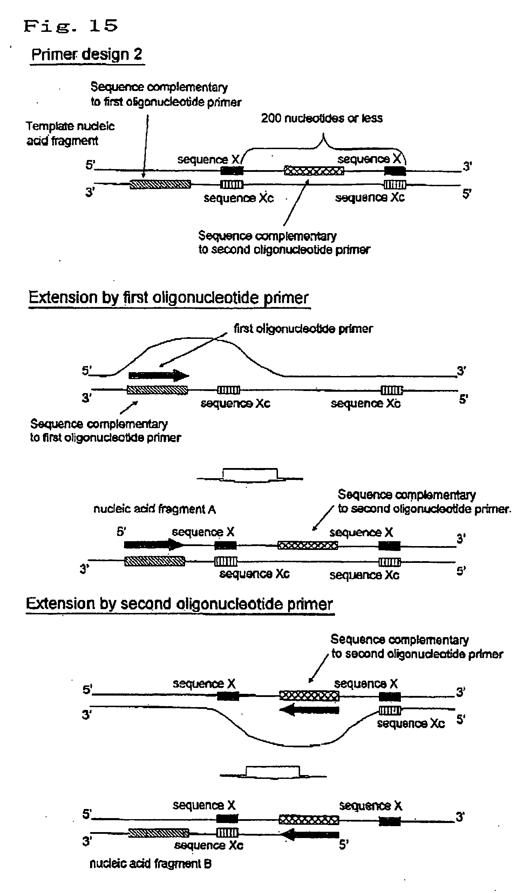

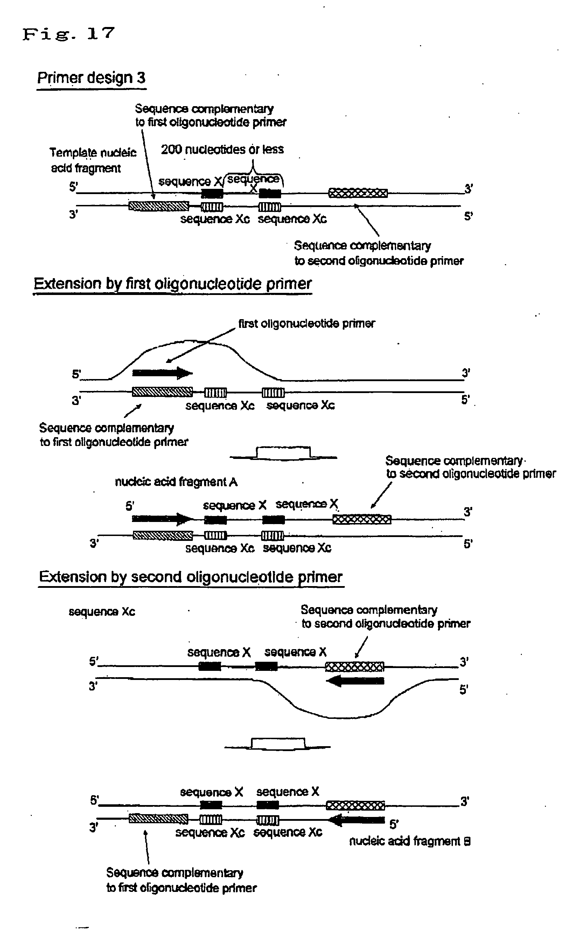

More specifically, the nucleic acid amplification method of the present invention is characterized in that a first oligonucleotide primer and a second oligonucleotide primer is designed in such a way that any one of the following requirements is satisfied.
(1) As to the region which contains two identical sequences X of serial 4 or more nucleotides within the region of 200 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer (Fig. 13).
(2) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X (Fig. 15).
(3) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X (Fig. 17).

Preferably, the first oligonucleotide primer and the second oligonucleotide primer arc designed in such a way that any one of the following requirements is satisfied.
(1) As to the region which contains two identical sequences X of serial 4 or more nucleotides within the region of 100 or less nucleotides, a first oligonucleotide primer is designed in a region which is sandwiched by a 5' terminal nucleotide or the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 100 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 100 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

Preferably, the first oligonucleotide primer and the second oligonucleotide primer are designed in such a way that any one of the following requirements is satisfied (1) As to the region which contains two identical sequences X of serial 4 or more nucleotides within the region of 60 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 60 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 60 or less nueleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

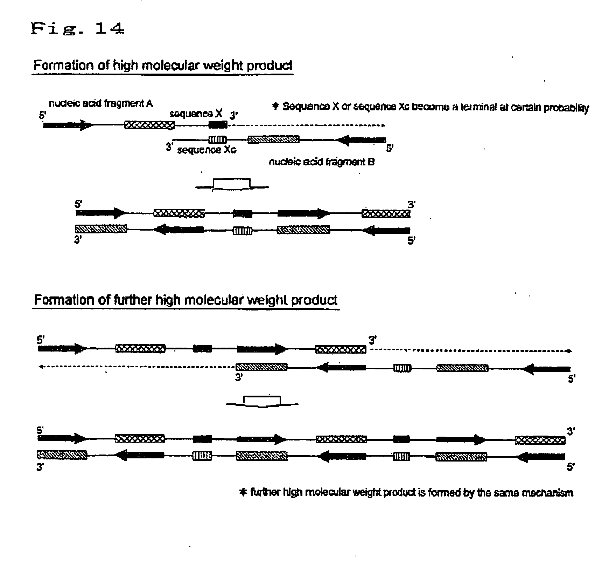

The outline of the nucleic acid amplification method (the above (1)) of the present invention is shown in Figs.13 and 14. A first oligonucleotide primer and a second oligonucleotide primer are annealed to a template nucleic acid strand, and the polymerase reaction is initiated from the 3' end of the oligonucleotide primer. At this moment, an amplified nucleic acid fragments (which is referred to as a nucleic acid fragment A) which contains the sequence X at 3' terminal is obtained as an amplified product of the polymerase reaction which is initiated from the first oligonucleotide primer.

In the same way, an amplified nucleic acid fragment (which is referred to as a nucleic acid fragment B) which contains the sequence Xc (which is complementary to the sequence X) at 3' terminal is obtained as an amplified product of the polymerase reaction which is initiated from the second oligonucleotide primer.

Then, the amplified nucleic acid fragment A is hybridized to the amplified nucleic acid fragment B, and elongation starts. Thus, a high molecular amplified nucleic acid fragment is synthesized.

Further, the amplified nucleic acid fragment A is hybridized, to the template nucleic acid fragment via the sequences X (sequence Xc), and elongation starts, and thus a high molecular amplified nucleic acid fragment is synthesized.

Further, the amplified nucleic acid fragment B is hybridized to the template nucleic acid fragment via the sequences X (sequence Xc), and elongation starts, and thus a high molecular amplified nucleic acid fragment is synthesized.

Any one or both of the first oligonucleotide primer and the second oligonucleotide primer can be annealed to the nucleic acid fragment A, the nucleic acid fragment B and the high molecular amplified nucleic acid fragment. Then, a polymerase reaction which is initiated from the 3' terminal progresses, and a replication reaction of a nucleic acid fragment occurs continuously. As a result, template nucleic acid fragment is amplified to a detectable level.

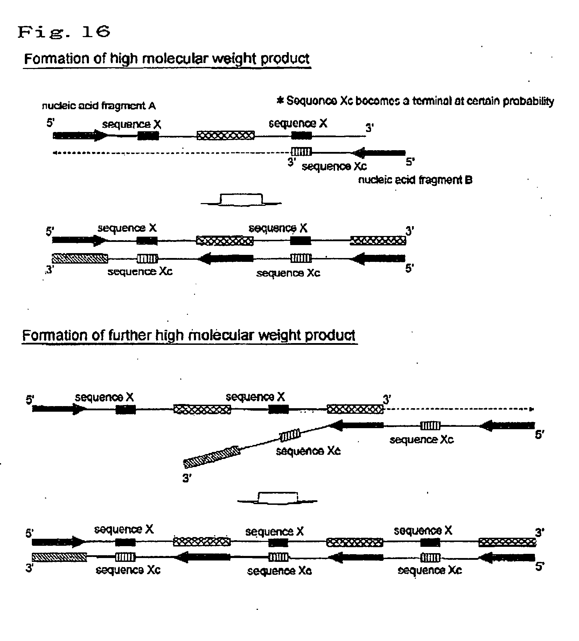

The outline of the nucleic acid amplification method (the above (2)) of the present invention is shown in Figs.15 and 16. A first oligonucleotide primer and a second oligonucleotide primer are annealed to a template nucleic acid strand, and the polymerase reaction is initiated from the 3' end of the oligonucleotide primer. At this moment, an amplified nucleic acid fragment (which is referred to as a nucleic acid fragment A) which contains at least two of the sequences X at 3' terminal is obtained as an amplified product of the polymerase reaction which is initiated from the first oligonucleotide primer.

In the same way, an amplified nucleic acid fragment (which is referred to as a nucleic acid fragment B) which contains the sequence Xc (which is complementary to the sequence X) at 3' terminal is obtained as an amplified product of the polymerase reaction which is initiated from the second oligonucleotide primer.

Then, the amplified nucleic acid fragment A is hybridized to the amplified nucleic acid fragment B, and elongation starts. Thus, a high molecular amplified nucleic acid fragment is synthesized.

Further, the amplified nucleic acid fragment A is hybridized to the template nucleic acid fragment via the sequences X (sequence Xc), and elongation starts, and thus a high molecular amplified nucleic acid fragment is synthesized.

Any one or both of the first oligonucleotide primer and the second oligonucleotide primer can be annealed to the nucleic acid fragment A, the nucleic acid fragment B and the high molecular amplified nucleic acid fragment. Then, a polymerase reaction which is initiated from the 3' terminal progresses, and a replication reaction of a nucleic acid fragment occurs continuously. As a result, template nucleic acid fragment is amplified to a detectable level.

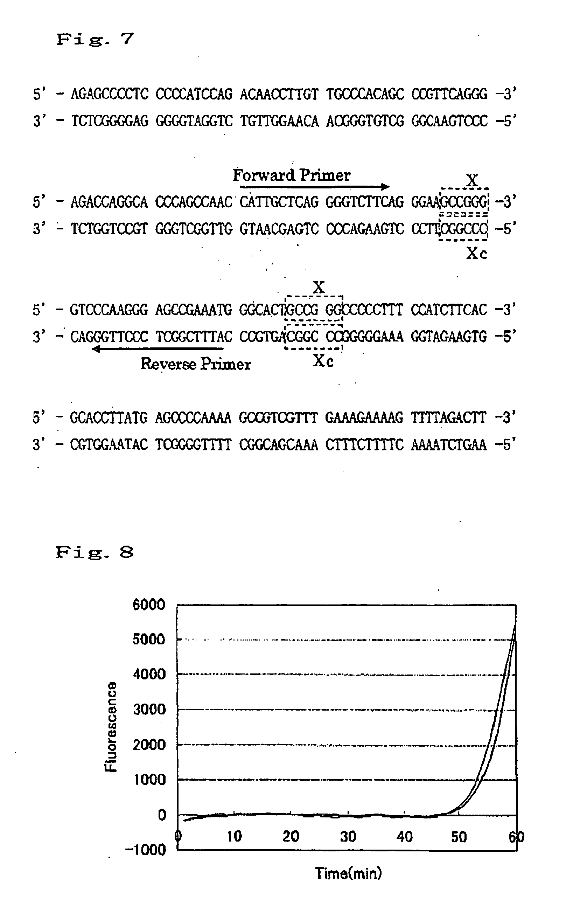

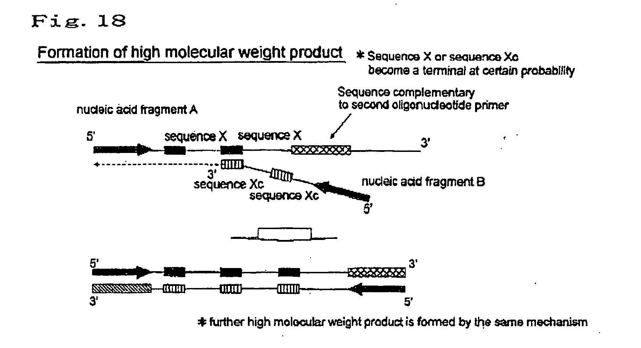

The outline of the nucleic acid amplification method (the above (3)) of the present invention is shown in Figs.7 and 18. A first oligonucleotide primer and a second oligonucleotide primer are annealed to a template nucleic acid strand, and the polymerase reaction is initiated from the 3' end of the oligonucleotide primer. At this moment, an amplified nucleic acid fragment (which is referred to as a nucleic acid fragment A) which contains at least two of the sequence X at 3' terminal is obtained as an amplified product of the polymerase reaction which is initiated from the first oligonucleotide primer.

In the same way, an amplified nucleic acid fragment (which is referred to as a nucleic acid fragment B) which contains at least two of the sequence Xc (which is complementary to the sequence X) at 3' terminal is obtained as an amplified product of the polymerase reaction which is initiated from the second oligonucleotide primer.

Then, the amplified nucleic acid fragment A is hybridized to the amplified nucleic acid fragment B, and elongation starts. Thus, a high molecular amplified nucleic acid fragment is synthesized.

Further, the amplified nucleic acid fragment A is hybridized to the template nucleic acid fragment via the sequences X (sequence Xc), and elongation starts, and thus a high molecular amplified nucleic acid fragment is synthesized.

Further, the amplified nucleic acid fragment B is hybridized to the template nucleic acid fragment via the sequences X (sequence Xc), and elongation starts, and thus a high molecular amplified nucleic acid fragment is synthesized.

Any one or both of the first oligonucleotide primer and the second oligonucleotide primer can be annealed to the nucleic acid fragment A, the nucleic acid fragment B and the high molecular amplified nucleic acid fragment. Then, a polymerase reaction which is initiated from the 3' terminal progresses, and a replication reaction of a nucleic acid fragment occurs continuously. As a result, template nucleic acid fragment is amplified to a detectable level.

Hereinafter, ingredients that are used in the present invention will be explained.

### (1) Deoxynucleotide triphosphate

Deoxynucleotide triphosphate is used as a substrate for an elongation reaction. Specifically, a mixture of dATP, dCTP, dGTP, and dTTP is preferably used. Deoxynucleotide triphosphate to be used herein may contain a dNTP analog (e.g., 7-deaza-dGTP).

Furthermore, deoxynucleotide triphosphate (dATP, dCTP, dGTP, or dTTP fixture) is at a final concentration ranging from 0.1 mM to 100 mM, preferably 0.75 mM to 3.0 mM, further preferably 1.0 mM to 2.0 mM, and particularly preferably 1.0 mM to 1.5 mM.

### (2) DNA polymerase

In the present invention, DNA polymerase is used. Preferably, polymerase capable of strand displacement (or having strand displacement activity) can be used as the DNA polymerase. In the description, "strand displacement activity" refers to activity by which strand displacement can be performed; that is, when DNA replication is performed based on a template nucleic acid sequence, strand displacement proceeds by replacement of DNA strands, so as to liberate a complementary strand that has annealed to the template strand. Specific examples of polymerase capable of strand displacement include, but arc not limited to, *Bacillus stearothermophilus-derived* 5'→3' exonuclease-deficient Bst. DNA polymerase, *Bacillus caldotenax-*derived 5'→3' exonuclease-deficient Bca DNA polymerase, and *Thermococcus litoralis-*derived 5'→3' exonuclease-deficient Vent. DNA polymerase. Such polymerase capable of strand displacement may be derived from nature or may be a genetically engineered recombinant protein.

### (3) Divalent cation

In the present invention, divalent cations may be used in response to metal requirements and the like regarding enzymes to be used herein. As divalent cations, magnesium salts or other metal salts can be used. For example, magnesium chloride, magnesium acetate, and magnesium sulfate can be used. Such a divalent cation is at a final concentration preferably ranging from 1 mM to 20 mM and further preferably ranging from 2 mM to 10 mM.

### (4) Surfactant

In the present invention, a surfactant may be added to a reaction solution. An advantagenous effect; that is, prevention of nonspecific nucleic acid amplification, is achieved via the use of a surfactant. Types of such surfactant that can be used in the present invention are not particularly limited, and may include the following: anionic surfactants such as alkylbenzene sulfonate, lauryl sulfate (SDS), octyl sulfosuccinate, and stearic acid soap;
nonionic surfactants such as sucrose fatty acid ester, sorbitan fatty acid ester, POE sorbitan fatty acid ester (e.g., Tween 20, Tween 40, Tween 60, Tween 80, and the like), fatty acid alkanol amide, POE alkyl ether (e.g., Brij35, Brij58, and the like), POE alkyl phenyl ether (e.g., Triton X-100, Triton X-114, Nonidet P40, and the like), nonylphenol, lauryl alcohol, polyethylene glycol, polyoxyethylene-polyoxypropylene block polymer, POE alkyl amine, and POE fatty acid bisphenyl ether;
cationic surfactants such as cetylpyridium chloride, lauryl dimethylbenzyl ammonium chloride, and stearyltrimethylammonium chloride.

The dose of such a surfactant is not particularly limited, as long as the effects of the present invention can be achieved and is preferably 0.01% or more, more preferably 0.05% or more, and more preferably 0.1% or more. The upper limit of the dose of such a surfactant is not particularly limited and is generally 10% or less, preferably 5% or less, and more preferably 1% or less.

Among the above surfactants, nonionic surfactants are preferably used. Among the nonionic surfactants, highly hydrophilic surfactants are preferred. The HLB value is preferably 12 or more, and further preferably 14 or more. Preferably, the upper limit of HLB is 20. Preferably, the value of HLB is 17 or less. More preferably, the value of HLB is 14 to 17. The surfactant is preferably selected from a polyoxyethylene sorbitan fatty acid ester-based surfactant and a polyoxyethylene alkyl ether-based surfactant. Among the polyoxyethylene sorbitan fatty acid ester, polyoxyethylene sorbitan mono fatty acid ester is preferred. Preferably the compound represented by the following formula can be used: 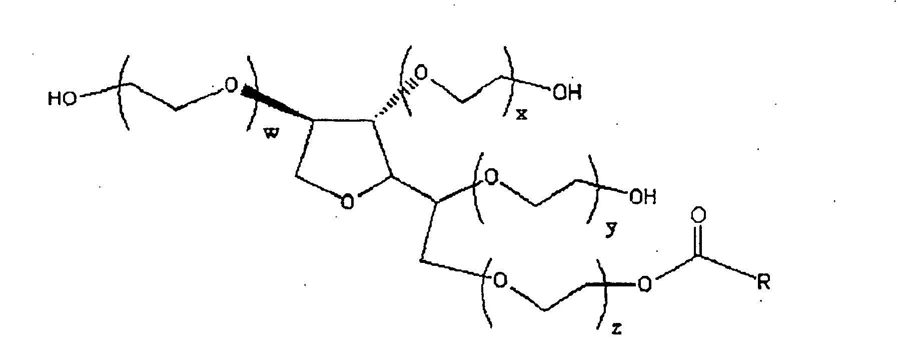 wherein x + y + z + w = 20, R is an alkyl group having a carbon number of 12 to 18.

The position of the alkyl group is not particularly limited, and the compound of the following structure can be preferably used. 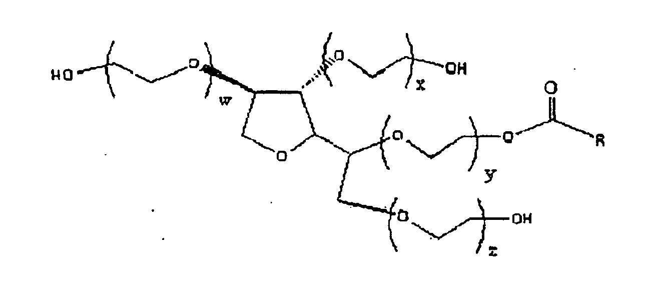 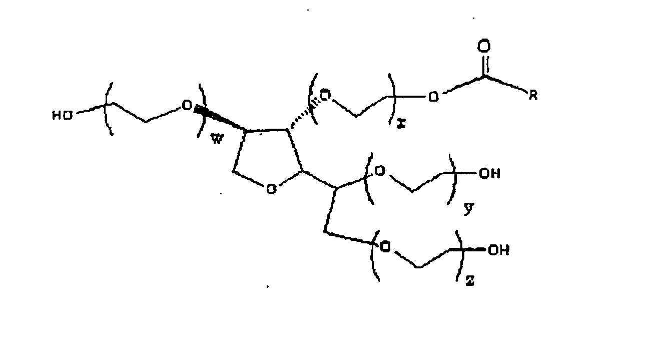 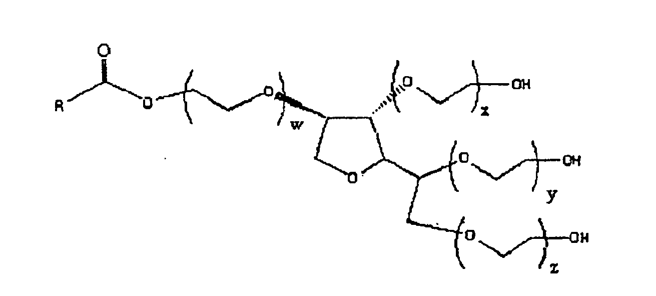 wherein x + y + z + w = 20, R is an alkyl group having a carbon number of 12 to 18.

Specific examples of such surfactants may include polyoxyethylene(20) sorbitan monolaurate, polyoxyethylene(20) sorbitan monopalmitate, polyoxyethylene(20) sorbitan monostearate, and polyoxyethylene(20) sorbitan monooleate (trade name: Tween 20, Tween 40, Tween 60, Tween 80, and the like). The dose of such surfactant is not particularly limited, and may be preferably 0.01% or more, more preferably 0.05% or more, and more preferably 0.1 % or more.

### (5) Oligonucleotide primer

The oligonucleotide primer to be used in the present invention has a nucleotide sequence substantially complementary to template DNA and has the 3' end from which DNA strand elongation is possible. Such oligonucleotide primer has a nucleotide sequence substantially complementary to template DNA, so that it can anneal to the template DNA. As an oligonucleotide primer to be used in the present invention, an oligonucleotide primer composed of a deoxyribonucleotide or a ribonucleotide can be used. Furthermore, an oligonucleotide primer containing a modified ribonucleotide or a modified deoxyribonucleotide may also be used herein.

For the aforementioned oligonucleotide primer, no complicated design such as those employed for conventional isothermal amplification reactions is required. An important feature of the present invention is resides in that isothermal amplification reactions can be carried out by using at least one set of primers which are used in the general PCR. Especially, these primers do not have a structure which forms a loop structure wherein 5' terminal is complementary to the region which was elongated from the 3'terminal as used in LAMP method. Namely, the consecutive region at 3'-terminal of the primer is complementary to the template nucleic acid. Further, the oligonudeotide primer has no complicated system where the primer is cleaved during the reaction and the cleaved 3' terminal serves as a synthesis origin, which is used in the SDA method or the ICAN method.

The aforementioned primers are designed in such a way that any one of the following requirements is satisfied.
(1) As to the region which contains two identical sequences X of serial 4 or more nucleotides within the region of 200 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 200 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

Preferably, the aforementioned primers are designed in such a way that any one of the following requirements is satisfied.
(1) As to the region which contains two identical sequences X of serial 4 or more nucleotides within the region of 100 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 100 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotide of the 5'-side sequence X and a 3' terminal nucleotide of the 3'-side sequence X.
(3) As to the region which contains two identical sequences X of serial 4 or more nucleotides within the region of 100 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

Preferably, the aforementioned primers are designed in such a way that any one of the following requirements is satisfied.
(1) As to the region which contains two identical sequences X of serial 4 or more nucleotides within the region of 60 or less nucleotides, a first oligonucleotide primer is designed in a region which is sandwiched by a 5' terminal nucleotide of the 5'-side sequence X and a 3' terminal nucleotide of the 3'-side sequence X, a second oligonucleotide primer is designed in a complementary sequence of a region which is sandwiched by a 5' terminal nucleotide of the 5'-side sequence X and a 3' terminal nucleotide of the 3'-side sequence X, and the second oligonucleotide primer is designed at a 5' side of a sequence which is complementary to the first oligonucleotide primer.
(2) As to the region which contains two identical sequences X of serial 4 or more nucleotides within the region of 60 or less nucleotides, a first oligonucleotide primer is designed in a region which is within 100 nucleotides at a 5' side of the 5'-side sequence X, and a second oligonucleotide primer is designed in a complementary sequence of a region which is sandwiched by a 5' terminal nucleotides of the 5'-side sequence X and a 3' terminal nucleotide of the 3'-side sequence X.
(3) As to the region which contains two identical sequences X of serial 4 or more nucleotides within the region of 60 or less nucleotides, a first oligonucleotide primer is designed in a region which is within 100 nucleotides at a 5' side of the 5'-side sequence X, and a second oligonucleotide primer is designed in a complementary sequence of a region which is within 100 nucleotides at a 3' side of the 3'-side sequence X.

Preferably, the sequence X and the sequence Xc comprise 5 or more nucleotide.

Preferably, the sequence X and the sequence Xc comprise 7 or more nucleotides.

The two sequences X of serial 4 or more nucleotides may be not completely identical. For example, if 4 or more nucleotide among 5 nucleotides, 5 or more nucleotide among 6 nucleotides, 5 or more nucleotide among 7 nucleotides, 6 or more nucleotide among 8 nucleotides, 7 or more nucleotide among 9 nucleotides, or 7 or more nucleotide among 10 nucleotides, are identical, amplification can occur in the same mechanism as in the case where the two sequences X are completely identical.

The length of an oligonucleotide primer is not particularly limited and generally ranges from approximately 10 to 100 nucleotides, preferably ranges from approximately 15 to 50 nucleotides, and further preferably ranges from approximately 15 to 40 nucleotides.

Oligonucleotide primers can be synthesized by the phosphoamidite method using a commercially available DNA synthesizer (e.g., Applied Biosystem Inc., DNA synthesizer 394).

The dose of an oligonucleotide primer is preferably 0.1 µM or more, further preferably 1 µM or more, and particularly preferably 1.5 µM or more.

### (6) Template nucleic acid fragment

In the present invention, template nucleic acid (DNA or RNA) may be any of genomic DNA, cDNA, synthetic DNA, mRNA, and total RNA. Nucleic acid that is prepared from a sample that may contain template nucleic acid may also be used. A sample that may contain template nucleic acid may also be directly used intact. Examples of the type of a sample containing template nucleic acid arc not particularly limited and include body fluids (e.g., whole blood, serum, urine, cerebrospinal fluid, seminal fluid, and saliva), tissues (e.g., cancer tissue), *in vivo* derived samples such as cell culture products, nucleic acid-containing samples such as viruses, bacteria, fungi, yeast, plants, and animals, samples that may be contaminated with microorganisms (e.g., foods), or samples in an environment such as soil or waste water. When nucleic acid is prepared from a sample described above, the preparation method therefor is not particularly limited. For example, methods known by persons skilled in the art can be used, including treatment using a surfactant, ultrasonication, purification using glass beads, and the like. Purification of nucleic acid from such a sample can be performed by phenol extraction, chromatography, gel electrophoresis, density gradient centrifugation, or the like.

For amplification of nucleic acid having an RNA-derived sequence, the method of the present invention can be implemented using cDNA as a template that is synthesized by a reverse transcription reaction using the RNA as a template. A primer to be used for a reverse transcription reaction may be a primer having a nucleotide sequence complementary to a specific template RNA, an oligo dT primer, or a primer having a random sequence. The length of a primer for reverse transcription preferably ranges from approximately 6 to 100 nucleotides and further preferably ranges from 9 to 50 nucleotides. Examples of an enzyme that can be used for a reverse transcription reaction are not particularly limited, as long as such an enzyme has activity of synthesizing cDNA with the use of template RNA and include avian myeloblastosis virus-derived reverse transcriptase (AMV RTase), moloney murine leukemia virus-derived reverse transcriptase (MMLV RTase), and rous associated virus 2 reverse transcriptase (RAV-2 RTase). Furthermore, strand displacement-type DNA polymerase that also has reverse transcription activity can also be used.

In the present invention, double-stranded DNA such as genomic DNA or a nucleic acid amplification fragment and single-stranded DNA such as DNA that is prepared from RNA via a reverse transcription reaction can be used as template DNAs. The above double-stranded DNA can be used for the method of the present invention after it has been denatured to single-stranded DNA or can also be used for the method of the present invention without performing such denaturation.

### (7) Melting temperature adjusting agent

A melting temperature adjusting agent can be added to a reaction solution to be used in the present invention. Specific examples of such a melting temperature adjusting agent include dimethyl sulfoxide (DMSO), betaine, formamide or glycerol, tetraalkyl ammonium salt, and a mixture of two or more types thereof. The dose for melting temperature adjustment is not particularly limited. In the case of DMSO, formamide, or glycerol, a melting temperature adjusting agent can be generally contained accounting for 10% or less of a reaction solution.

Betaine or tetraalkyl ammonium salt can be added at a concentration ranging from approximately 0.2 M to 3.0 M, preferably approximately 0.5 M to 1.5 M.

### (8) Buffer component

A reaction solution in the present invention can contain a buffer component. Examples of such a buffer component that can be used herein include, but are not particularly limited to, bicin, tricine, hepes, tris, and phosphate (e.g., sodium phosphate and potassium phosphate). The final concentration of such a buffer component ranges from 5 mM to 100 mM and particularly preferably ranges from 10 mM to 50 mM. Regarding pH, such a buffer component having pH generally ranging from 6.0 to 9.0 and particularly preferably ranging from 7.0 to 9.0 can be used, depending on optimum pH for an enzyme to be used for an amplification reaction.

### (9) Nucleic acid amplification method according to the present invention

Next, the nucleic acid amplification method according to the present invention will be described. According to the present invention, a reaction solution containing at least one type of deoxynucleotide triphosphate, at least one type of DNA polymerase, a divalent cation, at least two types of oligonucleotide primer, and a template nucleic acid fragment is incubated. Thus, a polymerase reaction that initiates from the 3' end of the primer is performed, so that the nucleic acid fragment can be amplified. Preferably in the present invention, a step of amplifying the nucleic acid fragment can be carried out substantially isothermally. A temperature for incubation of the reaction solution is preferably a room temperature or higher, more preferably 50°C or higher and more preferably 55°C or higher. For example, incubation can be performed at approximately 60°C. Preferably the temperature ranges from approximately 50°C to approximately 70°C and further preferably ranges from approximately 55°C to approximately 65°C, for example. In this case, nonspecific annealing of the primers is suppressed, specificity for DNA amplification is improved and the secondary structure of template DNA is dissolved. Hence, the elongation activity of DNA polymerase is also improved. The nucleic acid amplification method according to the present invention can be implemented substantially isothermally. "Isothermal or isothermally" in the present invention means that each step is performed at a substantially constant temperature without any significant changes in reaction temperature of each step.

In the present invention, the time required for substantially isothermal incubation of a reaction solution is not particularly limited, as long as a target nucleic acid fragment can be amplified. The time for incubation can be determined to be 5 minutes or more and 12 hours or less, for example. The time for incubation is preferably 5 minutes or more and 2 hours or less, more preferably 5 minutes or more and 60 minutes or less, and further preferably 5 minutes or more and 30 minutes or less. The time for incubation can also be 5 minutes or more and 15 minutes or less.

When a step of amplifying the nucleic acid fragment is carried out substantially isothermally, one of the advantages is that there is no need to raise or lower the temperature. Conventional PCR methods require to raise or lower the temperature. For example, such conventional PCR methods require a reaction apparatus such as a thermal cycler. However, the method of the present invention can be implemented with only an apparatus capable of maintaining a constant temperature.

### (10) Application of the nucleic acid amplification method according to the present invention

The nucleic acid amplification method according to the present invention can be used for nucleic acid detection, labeling, nucleotide sequence determination, detection of nucleotide mutation (including detection of single nucleotide polymorphism, for example), and the like. The nucleic acid amplification method of the present invention does not require the use of a reaction apparatus capable of performing temperature regulation. Thus, an amplification reaction can be performed according to the method using a large amount of a reaction solution.

Amplified products obtained by the use of the nucleic acid amplification method of the present invention can be detected by methods known by persons skilled in the art. For example, according to gel electrophoresis, gel is stained with ethidium bromide and then reaction products of a specific size can be detected. As detection systems for detection of amplified products, fluorescence polarization, immunoassay, fluorescent energy transfer, enzyme labels (e.g., peroxidase and alkaline phosphatase), fluorescent labels (e.g., fluorescein and rhodamine), chemiluminescence, bioluminescence, or the like can be used. Amplified products can also be detected using a labeled nucleotide labeled with biotin or the like. In such a case, biotin in an amplified product can be detected using fluorescence labeled avidin, enzyme-labeled avidin, or the like.

The present invention will be specifically described in the following examples. However, the examples are not intended to limit the present invention.

### Examples

### <Example 1> Nucleic acid amplification reaction

### (1) Preparation of nucleic acid sample solution containing target nucleic acid fragment

3.0 ng of Human Genomic DNA (produced by Clontech) was heated at 98°C for 3 minutes to be single-stranded, and a sequence in a β-actin gene was then amplified under the following conditions.

### <Primers>

Primers were designed using a β-actin gene as a target. Each primer sequence, is as shown below.
Primer (1) (Forward primer):
5'-GGGCATGGGTCAGAAGGATT-3' (SEQ ID NO: 1)
Primer (2) (Reverse primer):
5'-CCTCGTCGCCCACATAG-3' (SEQ ID NO: 2)

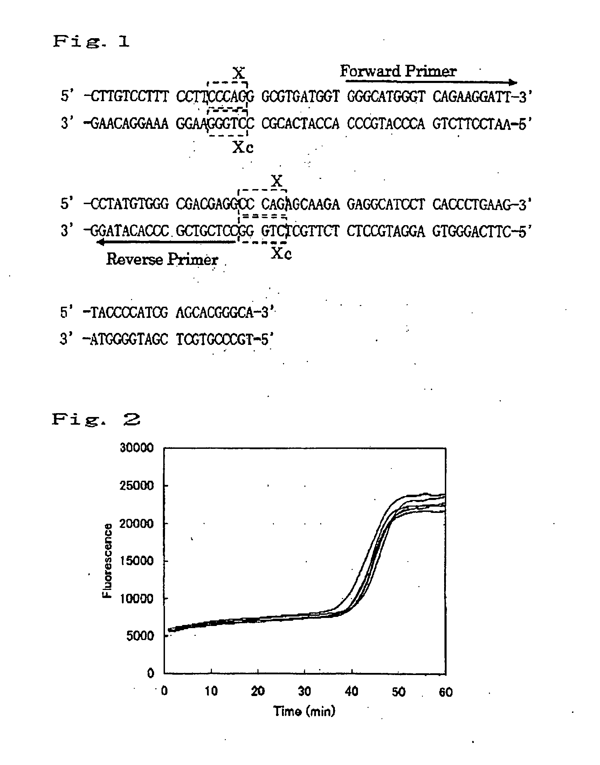

Details of the positional relationship of the aforementioned primers to the β-actin gene are as shown in Fig. 1.

In Fig. 1, the sequence X is 5'-CCCAG-3', and the sequences X are present at a position which are apart from each other by 54 nucleotides. The primer (1) is designed in a region which is sandwiched by the a 5' terminal nucleotide of the 5'-side sequence X and a 3' terminal nucleotide of the 3'-side sequence X, and the primer (2) is designed in a complementary sequence of a region which is sandwiched by a 5' terminal nucleotide of the 5'-side sequence X and a 3' terminal nucleotide of the 3'-side sequence X.

### (2) Nucleic acid amplification reaction

The amplification reaction was performed at 60°C for 60 minutes with the composition of a reaction solution shown below. Bst. DNA polymerase (NEB (New England Biolabs)) was used as an enzyme.

### <Composition of reaction solution>

| | |
|---|---|
| 10 x Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000 times) | 0.2 µL |
| 50 µM primer (1) | 0.64 µL |
| 50 µM primer (2) | 0.64 µL |
| Bst. Polymerase | 0.4 µL |
| Nucleic acid fragment sample | 0.4 µL |
| solution-obtained in (1) (3.0 ng) | |
| Purified water | 4.96 µL |
| | 10.0 µL |

### (3) Detection of amplified product

The amplification reaction in (2) above was carried out using a real-time fluorescence detection apparatus (Mx3000p, manufactured by Stratagene), and the fluorescence was detected. The results are shown in Fig. 2.

The time (Ct value) when an amount of fluorescence had reached 250 in the above graph was calculated using Mx3000p analysis software. The Ct value was 37.6 ± 1.1. minutes.

**[Table 1]**

| Ct (250) [min] |
|---|
| 38.8 |
| 37.3 |
| 38.2 |
| 37.2 |
| 35.7 |
| 38.1 |

### <Example 2> Electrophoresis of amplified products

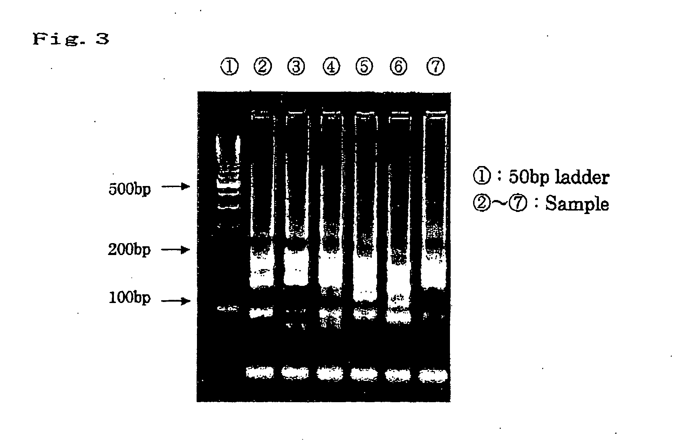

Electrophoresis was performed at 100 V for 60 minutes using 3 wt % agarose gel and 0.5 x TBE buffer (50 mM Tris, 45 mM Boric acid, and 0.5 mM EDTA, pH 8.4). The results are shown in Fig. 3. The electrophoretic patterns were almost uniform at N - 6, and thus it was found that the amplified products were obtained based on the same reaction mechanism.

### <Example 3> Cloning of amplified products

After completion of the electrophoresis, gel of the region of 200 bp or less was cut out, and DNA contained in the gel was then recovered using QIAEX II (manufactured by Qiagen).

The recovered DNA was incorporated into a vector using TOPO TA Cloning Kit (manufactured by Invitrogen), and *Escherichia coli* was then transformed with the vector. The transformed *Escherichia coli* was cultured in an LB medium containing ampicillin.

Thereafter, plasmid DNA was recovered from the cultured *Escherichia coli,* using QIAprep Miniprep (manufactured by Qiagen).

The recovered plasmid DNA was sequenced to determine the nucleotide sequence thereof. An M13 Reverse Primer was used as a primer.

### M13 Reverse Primer

5'-CAGGAAACAGCTATGAC-3' (SEQ ID NO: 3)

As a result of the sequencing, it was found that the following three types of amplified products were present.

### (1)

### (2)

### (3)

The chain lengths of the amplified products obtained by sequencing corresponded to the electrophoretic results as shown in Fig. 2.

The amplified product (1) was a region sandwiched between two primers.

The amplified product (2) was obtained as a result that products amplified by each primer were bound to one another via the sequence X (CCCAG) and the sequence Xc (CTGGG), and that the bound product was further amplified.

The amplified product (3) was obtained as a result that the amplified product (2) was bound to a product amplified by either one of the two primers via the sequence X (CCCAG) and the sequence Xc, (CTGGG), and that the bound product was further amplified.

### <Example 4> Nucleic acid amplification reaction

### (1) Preparation of nucleic acid sample solution containing target nucleic acid fragment

3.0 ng of Human Genomic DNA, (produced by Clontech) was heated at 98°C for 3 minutes to be single-stranded, and a sequence in a β3AR gene was then amplified under the following conditions.

### <Primers>

Primers were designed using a β3AR gene as a target. Each primer sequence is as shown below.
Primer (1) (Forward primer):
3'-ATCGTGGCCATCGCCT-3' (SEQ ID NO:7)
Primer (2) (Reverse primer):
5'CCAGCGAAGTCACGAAC-3' (SEQ ID NO:8)

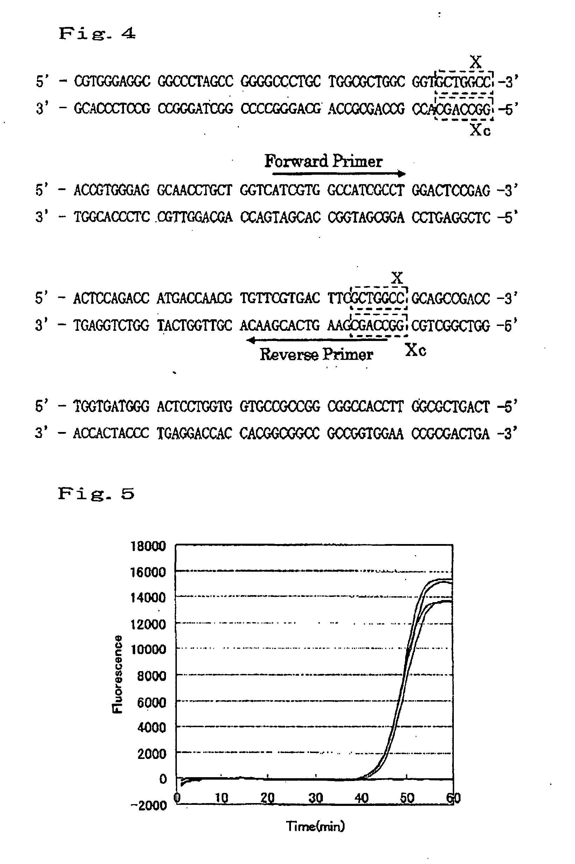

Details of the positional relationship of the aforementioned primers to the β3AR gene are as shown in Fig. 4.

In Fig. 4, the sequence X is 5'-GCTGGCC-3', and the sequences X are present at a position which are apart from each other by 90 nucleotides. The primer (1) is designed in a region which is sandwiched by the a 5' terminal nucleotide of the 5'-side sequence X and a 3' terminal nucleotide of the 3'-side sequence X, and the primer (2) is designed in a complementary sequence of a region which is sandwiched by a 5' terminal nucleotide of the 5'-side sequence X and a 3' terminal nucleotide of the 3'-side sequence X.

### (2) Nucleic acid amplification reaction

The amplification reaction was performed at 60°C for 60 minutes with the composition of a reaction solution shown below. Bst. DNA polymerase (NEB (New England Biolabs)) was used as an enzyme.

### <Composition of reaction solution>

| | |
|---|---|
| 10 x Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000 times) | 0.2 µL |
| 50 µM primer (1) | 0.64 µL |
| 50 µM primer (2) | 0.64 µL |
| Bst. Polymerase | 0.4 µL |
| Nucleic acid fragment sample | 0.4 µL |
| solution obtained in (1) (3.0 ng) | |
| Purified water | 4.96 µL |
| | 10.0 µL |

### (3) Detection of amplified product

The amplification reaction in (2) above was carried out using a real-time fluorescence detection apparatus (Mx3000p, manufactured by Stratagene), and the fluorescence was detected. The results are shown in Fig. 5.

The time (Ct value) when an amount of fluorescence had reached 250 in the above graph was calculated using Mx3000p analysis software. The Ct value was 41.2 ± 0.5 minutes.

**[Table 2]**

| Ct (250) [min] |
|---|
| 40.7 |
| 41.1 |
| 41.9 |
| 41.0 |

### <Example 5> Electrophoresis of amplified products

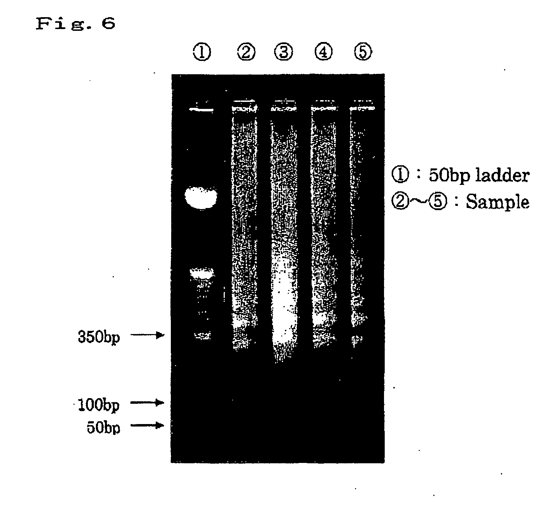

Electrophoresis was performed at 100 V for 60 minutes using 3 wt % agarose gel and 0.5 x TBE buffer (50 mM Tris, 45 mM Boric acid, and 0.5 mM EDTA, pH 8.4). The results are shown in Fig. 6. The electrophoretic patterns were almost uniform at N = 4, and thus it was found that the amplified products were obtained based on the same reaction mechanism.

### <Examples 6> Cloning of amplified products

After completion of the elechophoresis, gel of the region of 200 bp or less was cut out, and DNA contained in the gel was then recovered using QIAEX II (manufactured by Qiagen).

The recovered DNA was incorporated into a vector using TOPO TA Cloning Kit (manufactured by Invitrogen), and *Escherichia coli* was then transformed with the vector. The transformed *Escherichia coli* was cultured in an LB medium containing ampicillin.

Thereafter, plasmid DNA was recovered from the cultured *Escherichia coli,* using QIAprep Miniprep (manufactured by Qiagen).

The recovered plasmid DNA was sequenced to determine the nucleotide sequence thereof. An M13 Reverse Primer was used as a primer.
M13 Reverse Primer
5'-CAGGAAACAGCTATGAC-3' (SEQ ID NO: 3)

As a result of the sequencing, it was found that the following two types of amplified products were present.

### (1)

The chain lengths of the amplified products obtained by sequencing corresponded to the electrophoretic results as shown in Fig. 6.

The amplified product (1) was a region sandwiched between two primers.

The amplified product (2) was obtained as a result that products amplified by each primer were bound to one another via the sequence X (GCTGGCC) and the sequence Xc (GGCCAGC), and that the bound product was further amplified.

### <Example 7> Nucleic acid amplification reaction

### (1) Preparation of nucleic acid sample solution containing target nucleic acid fragment

3.0 ng of Human Genomic DNA (produced by Clontech) was heated at 98°C for 3 minutes to be single-stranded, and a sequence in chromosome 7 was then amplified under the following conditions.

### <Primers>

Primers were designed using a sequence in chromosome 7 as a target. Each primer sequence is as shown below.
Primer (5) (Forward primer):
5'-CATTGCTCAGGGGTCTTC-3'(SEQ ID NO:11)
Primer (6) (Reverse primer):
5'-ATTTCGGCTCCCTTGG-3'(SEQ ID NO:12)

Details of the positional relationship of the aforementioned primers to the sequence in chromosome 7 are as shown in Fig. 7.

In Fig. 7, the sequence X is 5'-GCCGGG-3', and the sequences X are present at a position which are apart from each other by 32 nucleotides. The primer (5) is designed in a region which is within 100 nucleotides at a 5' side of the 5'-side sequence X, and the primer (6) is designed in a complementary sequence of a region which is sandwiched by a 5' terminal nucleotide of the 5'-side sequence X and a 3' terminal nucleotide of the 3'-side sequence X.

### (2) Nucleic acid amplification reaction

The amplification reaction was performer at 60°C for 60 minutes with the composition of a reaction solution shown below. Bst. DNA polymerase (NEB (New England Biolabs)) was used as an enzyme.

### <Composition of reaction solution>

| | |
|---|---|
| 10 x Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000 times) | 0.2 µL |
| 50 µM primer (5) | 0.64 µL |
| 50 µM primer (6) | 0.64 µL |
| Bst. Polymerase | 0.4 µL |
| Nucleic acid fragment sample | 0.4 µL |
| solution obtained in (1) (3.0 ng) | |
| Purified water | 4.96 µL |
| | 10.0 µL |

### (3) Detection of amplified product

The amplification reaction in (2) above was carried out using a real-time fluorescence detection apparatus (Mx3000p, manufactured by Stratagene), and the fluorescence was detected. The results are shown in Fig. 8.

The time (Ct value) when an amount of fluorescence had reached 250 in the above graph was calculated using Mx3000p analysis software. The Ct value was 50.1 ± 0.4 minutes.

**[Table 3]**

| Ct (250) [min] |
|---|
| 49.8 |
| 50.4 |

### <Examples 8> Electrophoresis of amplified products

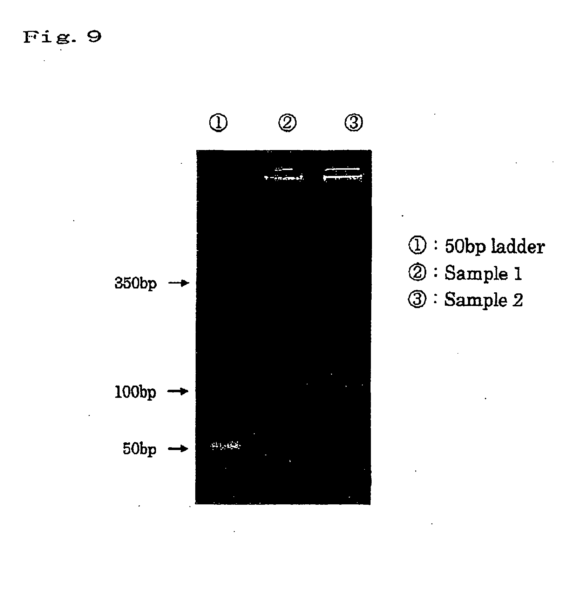

Electrophoresis was performed at 100 V for 60 minutes using 3 wt % agarose gel and 0.5 x TBE buffer (50 mM Tris, 45 mM Boric acid, and 0.5 mM EDTA, pH 8.4). The results are shown in Fig. 9. The electrophoretic patterns were almost uniform at N = 2, and thus it was found that the amplified products were obtained based on the same reaction mechanism.

### <Example 9> Nucleic acid amplification reaction

### (1) Preparation of nucleic acid sample solution containing target nucleic acid fragment

3.0 ng of Human Genomic DNA (produced by Clontech) was heated at 98°C for 3 minutes to be single-stranded, and a sequence in chromosome 7 was then amplified under the following conditions.

### <Primers>

Primers were designed using a sequence in chromosome 7 as a target. Each primer sequence is as shown below.
Primer (5) (Forward primer):
5'-CATTGCTCAGGGGTCTTC-3' (SEQ ID NO:11)
Primer (7) (Reverse primer):
5'-GGGCTCATAAGGTGCGTG-3' (SEQ ID NO:13)

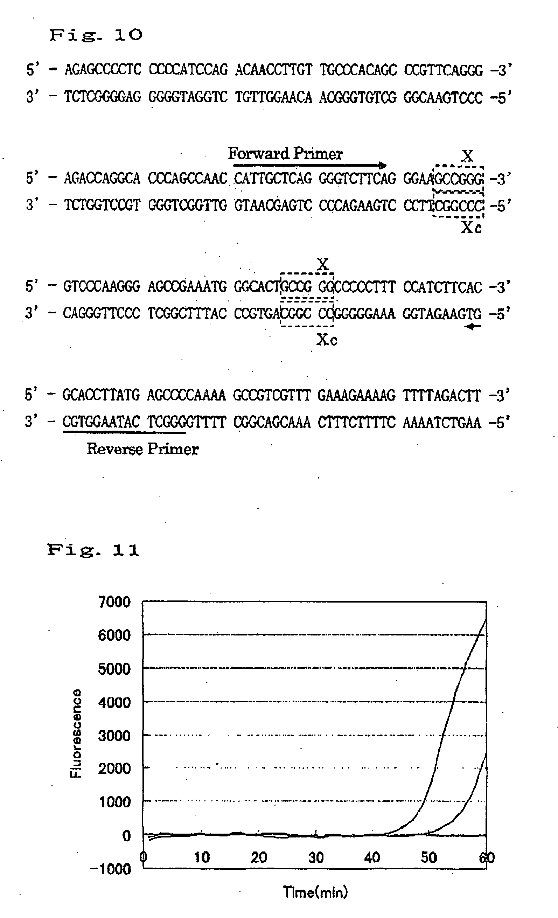

Details of the positional relationship of the aforementioned primers to the sequence in chromosome 7 are as shown in Fig. 10.

In Fig. 7, the sequence X is 5'-GCCGGG-3', and the sequences X are present at a position which are apart from each other by 32 nucleotides. The primer (5) is designed in a region which is within 100 nucleotides at a 5' side of the 5'-side sequence X, and the primer (7) is designed in a complementary sequence of a region which is within 100 nucleotides at a 3' side of the 3'-side sequence X.

### (2) Nucleic acid amplification reaction

The amplification reaction was performed at 60°C for 60 minutes with the composition of a reaction solution shown below. Bst. DNA polymerase (NEB (New England Biolabs)) was used as an enzyme.

### <Composition of reaction solution>

| | |
|---|---|
| 10 x Bst Buffer (DF) | 1.0 µL |
| 100 mM MgSO4 | 0.6 µL |
| 10% (v/v) Tween 20 | 0.1 µL |
| 100% DMSO | 0.5 µL |
| 25 mM dNTP each | 0.56 µL |
| SYBR Green I (2000 times) | 0.2 µL |
| 50 µM primer (5) | 0.64 µL |
| 50 µM primer (7) | 0.64 µL |
| Bst. Polymerase | 0.4 µL |
| Nucleic acid fragment sample | 0.4 µL |
| solution obtained in (1) (3.0 ng) | |
| Purified water | 4.96 µL |
| | 10.0 µL |

### (3) Detection of amplified product

The amplification reaction in (2) above was carried out using a real-time fluorescence detection apparatus (Mx3000p, manufactured by Stratagene), and the fluorescence was detected. The results are shown in Fig. 11

The time (Ct value) when an amount of fluorescence had reached 250 in the above graph was calculated using Mx3000p analysis software. The Ct value was 49.0 ± 5.0 minutes.

**[Table 4]**

| Ct (250) [min] |
|---|
| 52.5 |
| 45.5 |

### <Example 10> Electrophoresis of amplified products

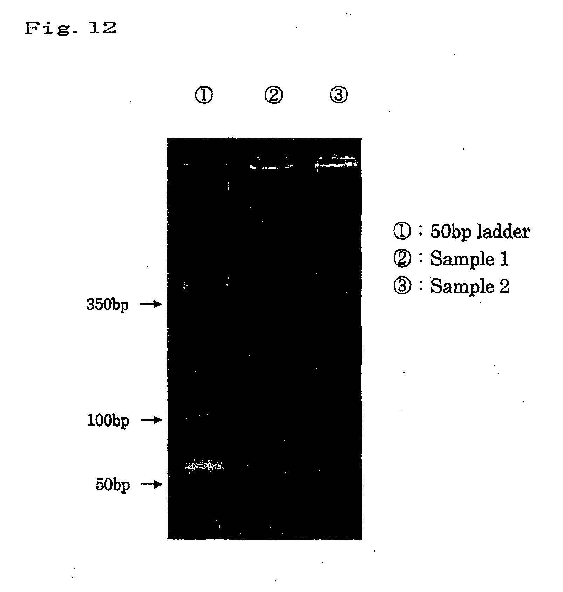

Electrophoresis was performed at 100 V for 60 minutes using 3 wt % agarose gel and 0.5 x TBE buffer (50 mM Tris, 45 mM Boric acid, and 0.5 mM EDTA, pH 8.4). The results are shown in Fig. 12. The electrophoretic patterns were almost uniform at N = 2, and thus it was found that the amplified products were obtained based on the same reaction mechanism.

## Claims

1. A method of designing a first and second oligonucleotide primer for an isothermal nucleic acid amplification reaction using at least one type of DNA polymerase having strand displacement activity, which primers are suitable for amplifying a template nucleic acid or a part thereof which contains a first and second sequence X, wherein the first and second sequence X have two identical sequences of 4 or more consecutive nucleotides and lie within 200 nucleotides or less of the template nucleic acid or fragment thereof, wherein the first sequence X lies 5' to the second sequence X, and wherein the primers satisfy any one of the requirements selected from (a) to (c)
(a) a first oligonucleotide primer is designed in a region lying between the 5' terminal nucleotide of the first sequence X and the 3' terminal nucleotide of the second sequence X, a second oligonucleotide primer is designed in the complementary sequence to the said region, such that the second oligonucleotide primer lies 5' to the sequence which is complementary to the first oligonucleotide primer, as judged on the complementary strand;
(b) a first oligonucleotide primer is designed in a region which is within 100 nucleotides 5' of the first sequence X, and a second oligonucleotide primer is designed in a complementary sequence of a region which is between the 5' terminal nucleotide of the first sequence X and the 3' terminal nucleotide of the second sequence X;
(c) a first oligonucleotide primer is designed in a region which is within 100 nucleotides 5' to the first sequence X, and a second oligonucleotide primer is designed in a complementary sequence of a region which is within 100 nucleotides 3' to the second sequence X.

2. The method of claim 1, wherein the sequence X and a sequence Xc which is complementary to the sequence X, comprise 5 or more nucleotides.

3. The method of claim 1, wherein the sequence X and a sequence Xc which is complementary to the sequence X, comprise 7 or more nucleotides.

4. The method of claim 1 wherein the oligonucleotide primers are substantially complementary to portions of the template nucleic acid fragment.

5. The method of claim 1, wherein only the 3' terminal region of the oligonucleotide primers is substantially complementary to portions of the template nucleic acid fragment.

6. The method of claim 5, wherein the oligonucleotide primers are substantially complementary to only consecutive 1 site of the template nucleic acid fragments.

7. The method of claim 1, wherein at least one type of the DNA polymerase having strand displacement activity is polymerase selected from the group consisting of Bacillus stearothermophilus-derived 5' → 3' exonuclease-deficient Bca DNA polymerase, and Thermococcus litoralis-derived 5' → 3' exonuclease-deficient Vent. DNA polymerase.

8. The method of claim 1, wherein the isothermal nucleic acid amplification reaction is carried out substantially isothermally at a temperature of a room temperature to 100°C.

9. An isothermal nucleic acid amplification method which comprises performing incubation of a reaction solution containing at least one type of deoxynucleotide triphosphate, at least one type of DNA polymerase having strand displacement activity, at least a first and second type of oligonucleotide primer, and the nucleic acid fragment as a template so as to perform a polymerase reaction that initiates from the 3' end of the primer and thus amplifying the nucleic acid fragment;
comprising performing the method according to any one of claims 1 to 8.

10. The method of claim 9, wherein the reaction solution contains at least 0.05 % or more surfactant.

11. The method of claim 10, wherein the surfactant is a non-ionic surfactant.

12. The method of claim 11, wherein the non-ionic surfactant is selected from among a polyoxyethylene sorbitan fatty acid ester-based surfactant, a polyoxyethylene alkyl phenol ether-based surfactant, and a polyoxyethylene alkyl ether-based surfactant.

13. The method of claim 9, wherein the reaction solution contains a divalent cation.

14. The method of claim 9, wherein the reaction solution further contains a melting temperature adjusting agent.

15. The method of claim 14, wherein the melting temperature adjusting agent is dimethyl sulfoxide, betaine, formamide, or glycerol, or a mixture of two or more types thereof.

16. The method of claim 9, wherein the reaction solution contains each deoxynucleotide triphosphate of 1.0 mM to 100 mM.

17. The method of claim 9, wherein the reaction solution contains each oligonucleotide primer of 1 µM to 100 µM.

## Patentansprüche

1. Verfahren zum Entwerfen eines ersten und eines zweiten Oligonukleotid-Primers für eine isothermische Nukleinsäure-Vervielfältigungsreaktion unter Verwendung von mindestens einer Art von DNA-Polymerase mit Strangverdrängungsaktivität, wobei die Primer geeignet sind, eine Vorlage-Nukleinsäure oder einen Teil davon zu vervielfältigen, die/der eine erste und eine zweite Sequenz X enthält, wobei die erste und die zweite Sequenz X zwei identische Sequenzen von 4 oder mehr aufeinanderfolgenden Nukleotiden aufweisen und innerhalb von 200 Nukleotiden oder weniger auf der Vorlage-Nukleinsäure oder dem Fragment davon liegen, wobei die erste Sequenz X auf der 5'-Seite relativ zur zweiten Sequenz X liegt und wobei die Primer eine der Anforderungen, ausgewählt aus (a) bis (c), erfüllt:
(a) ein erster Oligonukleotid-Primer wird in einer Region entworfen, die zwischen dem terminalen Nukleotid am 5'-Ende der ersten Sequenz X und dem terminalen Nukleotid am 3'-Ende der zweiten Sequenz X liegt, und ein zweiter Oligonukleotid-Primer wird in der komplementären Sequenz zu dieser Region entworfen, so dass der zweite Oligonukleotid-Primer auf der 5'-Seite der Sequenz liegt, die komplementär zum ersten Oligonukleotid-Primer ist, ausgehend vom komplementären Strang beurteilt;
(b) ein erster Oligonukleotid-Primer wird in einer Region entworfen, die innerhalb von 100 Nukleotiden 5' von der ersten Sequenz X ist, und ein zweiter Oligonukleotid-Primer wird in einer komplementären Sequenz einer Region entworfen, die zwischen dem terminalen Nukleotid am 5'-Ende der ersten Sequenz X und dem terminalen Nukleotid am 3'-Ende der zweiten Sequenz X ist;
(c) ein erster Oligonukleotid-Primer wird in einer Region entworfen, die innerhalb von 100 Nukleotiden 5' von der ersten Sequenz X ist, und ein zweiter Oligonukleotid-Primer wird in einer komplementären Sequenz zu der Region entworfen, die innerhalb von 100 Nukleotiden 3' zu der zweiten Sequenz X ist.

2. Verfahren nach Anspruch 1, wobei die Sequenz X und eine Sequenz Xc, die komplementär zu der Sequenz X ist, 5 oder mehr Nukleotide umfassen.

3. Verfahren nach Anspruch 1, wobei die Sequenz X und eine Sequenz Xc, die komplementär zu der Sequenz X ist, 7 oder mehr Nukleotide umfassen.

4. Verfahren nach Anspruch 1, wobei die Oligonukleotid-Primer im wesentlichen komplementär zu Abschnitten des Vorlage-Nukleinsäurefragments sind.

5. Verfahren nach Anspruch 1, wobei nur die 3'-terminale Region des Oligonukleotid-Primers im wesentlichen komplementär zu Abschnitten des Vorlage-Nukleinsäurefragments ist.

6. Verfahren nach Anspruch 5, wobei die Oligonukleotid-Primer nur zu einer fortlaufenden Stelle des Vorlage-Nukleinsäurefragments im wesentlichen komplementär sind.

7. Verfahren nach Anspruch 1, wobei mindestens eine Art der DNA-Polymerase mit Strangverdrängungsaktivität aus der Gruppe ausgewählt wird, die aus von Bacillus stearothermophilus abgeleiteter 5' → 3'-Exonukleasedefizienter Bca-DNA-Polymerase und von Thermococcus litoralis abgeleiteter 5' → 3'-Exonukleasedefizienter Vent.-DNA-Polymerase ausgewählt wird.

8. Verfahren nach Anspruch 1, wobei die isothermische Nukleinsäure-Vervielfältigungsreaktion im wesentlichen bei einer Temperatur von Raumtemperatur bis 100°C durchgeführt wird.

9. Isothermische Nukleinsäure-Vervielfältigungsreaktion, umfassend das Inkubieren einer Reaktionslösung, die mindestens eine Art von Desoxynukleotidtriphosphat, mindestens eine Art von DNA-Polymerase mit Strangverdrängungsaktivität, mindestens eine erste und eine zweite Art von Oligonukleotid-Primer und ein Nukleinsäurefragment als Vorlage enthält, um eine Polymerasereaktion durchzuführen, die am 3'-Ende des Primers beginnt und hierdurch Vervielfältigen des Nukleinsäurefragments; umfassend die Durchführung des Verfahrens nach einem der Ansprüche 1 bis 8.

10. Verfahren nach Anspruch 9, wobei die Reaktionslösung mindestens 0,05 % oder mehr Tensid enthält.

11. Verfahren nach Anspruch 10, wobei das Tensid ein nicht-ionisches Tensid ist.

12. Verfahren nach Anspruch 11, wobei das nicht-ionische Tensid aus einem PolyoxyethylensorbitanFettsäureester-basierten Tensid, einem Polyoxyethylenalkylphenolether-basierten Tensid und einem Polyoxyethylenalkylether-basierten Tensid ausgewählt wird,

13. Verfahren nach Anspruch 9, wobei die Reaktionslösung ein zweiwertiges Kation enthält.

14. Verfahren nach Anspruch 9, wobei die Reaktionslösung ferner ein Schmelztemperatur-Regulierungsmittel enthält.

15. Verfahren nach Anspruch 14, wobei das Schmelztemperatur-Regulierungsmittel Dimethylsulfoxid, Betain, Formamid, Glycerin oder eine Mischung von zwei oder mehr Arten davon ist.

16. Verfahren nach Anspruch 9, wobei die Reaktionslösung jedes Desoxynukleotidtriphosphat in einer Menge zwischen 1,0 und 100 mM enthält.

17. Verfahren nach Anspruch 9, wobei die Reaktionslösung jeden Oligonukleotid-Primer in einer Menge von 1 bis 100 µM enthält.

## Revendications

1. Procédé de conception d'une première et d'une deuxième amorce oligonucléotidique pour une réaction isotherme d'amplification d'acide nucléique utilisant au moins un type d'ADN polymérase ayant une activité de déplacement de brins, lesquelles amorces sont appropriées pour amplifier un acide nucléique matrice ou une partie de celui-ci qui contient une première et une deuxième séquence X, dans lequel la première et la deuxième séquence X ont deux séquences identiques de 4 nucléotides consécutifs ou plus et sont à 200 nucléotides ou moins de l'acide nucléique matrice ou du fragment de celui-ci, dans lequel la première séquence X est en 5' par rapport à la deuxième séquence X, et dans lequel les amorces satisfont à l'une quelconque des exigences choisies parmi (a) à (c)
(a) une première amorce oligonucléotidique est conçue dans une région entre le nucléotide terminal 5' de la première séquence X et le nucléotide terminal 3' de la deuxième séquence X, une deuxième amorce oligonucléotidique est conçue dans la séquence complémentaire de ladite région, de telle sorte que la deuxième amorce oligonucléotidique est en 5' par rapport à la séquence qui est complémentaire de la première amorce oligonucléotidique, ainsi qu'il est jugé sur le brin complémentaire ;
(b) une première amorce oligonucléotidique est conçue dans une région qui est à 100 nucléotides en 5' par rapport à la première séquence X, et une deuxième amorce oligonucléotidique est conçue dans une séquence complémentaire d'une région qui est entre le nucléotide terminal 5' de la première séquence X et le nucléotide terminal 3' de la deuxième séquence X ;
(c) une première amorce oligonucléotidique est conçue dans une région qui est à 100 nucléotides en 5' par rapport à la première séquence X, et une deuxième amorce oligonucléotidique est conçue dans une séquence complémentaire d'une région qui est à 100 nucléotides en 3' par rapport à la deuxième séquence X.

2. Procédé selon la revendication 1, dans lequel la séquence X et une séquence Xc qui est complémentaire de la séquence X comprennent 5 nucléotides ou plus.

3. Procédé selon la revendication 1, dans lequel la séquence X et une séquence Xc qui est complémentaire de la séquence X comprennent 7 nucléotides ou plus.

4. Procédé selon la revendication 1, dans lequel les amorces oligonucléotidiques sont sensiblement complémentaires de parties du fragment d'acide nucléique matrice.

5. Procédé selon la revendication 1, dans lequel seule la région terminale 3' des amorces oligonucléotidiques est sensiblement complémentaire de parties du fragment d'acide nucléique matrice.

6. Procédé selon la revendication 5, dans lequel les amorces oligonucléotidiques ne sont sensiblement complémentaires que d'un site consécutif des fragments d'acide nucléique matrice.

7. Procédé selon la revendication 1, dans lequel au moins un type de l'ADN polymérase ayant une activité de déplacement de brins est une polymérase choisie parmi le groupe consistant en une ADN polymérase Bca déficitaire en exonucléase 5' → 3' dérivée de *Bacillus stearothermophilus,* et une ADN polymérase Vent déficitaire en exonucléase 5' → 3' dérivée de *Thermococcus litoralis.*

8. Procédé selon la revendication 1, dans lequel la réaction isotherme d'amplification d'acide nucléique est conduite de façon sensiblement isotherme à une température d'une température ambiante à 100°C.

9. Procédé isotherme d'amplification d'acide nucléique qui comprend l'exécution d'une incubation d'une solution réactionnelle contenant au moins un type de désoxynucléotide triphosphate, au moins un type d'ADN polymérase ayant une activité de déplacement de brins, au moins un premier et un deuxième type d'amorce oligonucléotidique, et le fragment d'acide nucléique comme une matrice de façon à effectuer une réaction par polymérase qui s'initie à partir de l'extrémité 3' de l'amorce, et amplifiant ainsi le fragment d'acide nucléique ;
comprenant l'exécution du procédé selon l'une quelconque des revendications 1 à 8.

10. Procédé selon la revendication 9, dans lequel la solution réactionnelle contient au moins 0,05% ou plus d'un agent tensioactif.

11. Procédé selon la revendication 10, dans lequel l'agent tensioactif est un agent tensioactif non ionique.

12. Procédé selon la revendication 11, dans lequel l'agent tensioactif non ionique est choisi parmi un agent tensioactif à base d'ester d'acide gras de polyoxyéthylène sorbitan, un agent tensioactif à base d'éther alkylphénolique de polyoxyéthylène et un agent tensioactif à base d'éther alkylique de polyoxyéthylène.

13. Procédé selon la revendication 9, dans lequel la solution réactionnelle contient un cation divalent.

14. Procédé selon la revendication 9, dans lequel la solution réactionnelle contient en outre un agent d'ajustement de la température de fusion.

15. Procédé selon la revendication 14, dans lequel l'agent d'ajustement de la température de fusion est le diméthylsulfoxyde, la bétaïne, le formamide, ou le glycérol, ou un mélange de deux types de ceux-ci ou plus.

16. Procédé selon la revendication 9, dans lequel la solution réactionnelle contient chaque désoxynucléotide triphosphate de 1,0 mM à 100 mM.

17. Procédé selon la revendication 9, dans lequel la solution réactionnelle contient chaque amorce oligonucléotidique de 1 µM à 100 µM.


### SEQUENCE LISTING

<110> FUJIFILM Corporation
<120> Nucleic Acid Amplification Method
<130> FA8154A
<160> 13
<210> 1
   <211> 20
   <212> DNA
   <213> Artificial Sequence
<220>
   <223> Description of Artificial Sequence: Synthetic DNA
<400> 1
   gggcatgggt cagaaggatt 20
<210> 2
   <211> 17
   <212> DNA
   <213> Artificial Sequence
<220>
   <223> Description of Artificial Sequence: Synthetic DNA
<400> 2
   cctcgtcgcc cacatag 17
<210> 3
   <211> 17
   <212> DNA
   <213> Artificial Sequence
<220>
   <223> Description of Artificial Sequence: Synthetic DNA
<400> 3
   caggaaacag ctatgac 17
<210> 4
   <211> 38
   <212> DNA
   <213> Artificial Sequence
<220>
   <223> Description of Artificial Sequence: PCR product
<400> 4
   gggcatgggt cagaaggatt cctatgtggg cgacgagg 38
<210> 5
   <211> 92
   <212> DNA
   <213> Artificial Sequence
<220>
   <223> Description of Artificial Sequence: PCR product
<400> 5 <210> 6
   <211> 146
   <212> DNA
   <213> Artificial Sequence
<220>
   <223> Description of Artificial Sequence: PCR product
<400> 6
<210> 7
   <211> 16
   <212> DNA
   <213> Artificial Sequence
<220>
   <223> Description of Artificial Sequence: Synthetic DNA
<400> 7
   atcgtggcca tcgcct 16
<210> 8 .
   <211> 17
   <212> DNA
   <213> Artificial Sequence
<220>
   <223> Description of Artificial Sequence: Synthetic DNA
<400> 8
   ccagcgaagt cacgaac 17
<210> 9
   <211> 64
   <212> DNA
   <213> Artificial Sequence
<220>
   <223> Description of Artificial Sequence: PCR product
<400> 9
   atcgtggcca tcgcctggac tccgagacac cagaccatga ccaacgtgtt cgtgacttcg 60
   ctgg 64
<210> 10
   <211> 153
   <212> DNA
   <213> Artificial Sequence
<220>
   <223> Description of Artificial Sequence: PCR product
<400> 10
   atcgtggcca tcgcctggac tccgagacac cagaccatga ccaacgtgtt cgtgacttcg 60
   ctgggcaccg tgggaggcaa cctgctggtc atcgtggcca tcgcctggac tccgagacac 120
   cagaccatga ccaacgtgtt cgtgacttcg ctg 153
<210> 11
   <211> 18
   <212> DNA
   <213> Artificial Sequence
<220>
   <223> Description of Artificial Sequence: Synthetic DNA
<400> 11
   cattgctcag gggtcttc 18
<210> 12
   <211> 16
   <212> DNA
   <213> Artificial Sequence
<220>
   <223> Description of Artificial Sequence: Synthetic DNA
<400> 12
   atttcggctc ccttgg 16
<210> 13
   <211> 18 <212> DNA
   <213> Artificial Sequence
<220>
   <223> Description of Artificial Sequence: Synthetic DNA
<400> 13
   gggctcataa ggtgcgtg 18